# EUROPÄISCHE PATENTANMELDUNG

(11) **EP 3 613 811 A1**
(43) Veröffentlichungstag der Anmeldung: **26.02.2020**
(21) Anmeldenummer: 18190262.8
(22) Anmeldetag: 22.08.2018
(51) Int. Cl.: C09C 1/00, C09D 5/36, C09D 11/037, A61K 8/02

(54) **CHAMPAGNER-FARBTON METALLEFFEKTPIGMENTE**

(71) Anmelder: Schlenk Metallic Pigments GmbH, 91154 Roth (DE)
(72) Erfinder: Binder, Yvonne, 91186 Büchenbach (DE); Huber, Adalbert, 64625 Bensheim (DE); Graser, Daniel, 91126 Schwabach (DE)
(74) Vertreter: Müller-Boré & Partner Patentanwälte PartG mbB

(57) **Zusammenfassung**

Die vorliegende Erfindung betrifft Metalleffektpigmente mit hohem Deckvermögen und dünner Eisenoxidschicht zur Erzeugung eines Champagner-Farbtons.

## Beschreibung

Die vorliegende Erfindung betrifft Metalleffektpigmente mit hohem Deckvermögen und dünner Eisenoxidschicht zur Erzeugung eines Champagner-Farbtons.

Es sind bisher keine vergleichbaren Metalleffekt- oder Perlglanzpigmente mit hohem Deckvermögen, einem gelblich-beigen Farbton mit einem Bunttonwinkel (hᵤᵥ) im Bereich von 50-65 (Champagner-Farbton) sowie einer Sättigung (Sᵤᵥ) im Bereich von 0,2-0,3 (bei einem ΔE45° von ≤ 3) bekannt. Aktuell auf dem Markt befindliche Pigmente mit ähnlichen Farbtönen wie z.B. ALOXAL® der Firma Eckart GmbH oder Iriodin 9602 der Firma Merck weisen ein z.T. nicht zufriedenstellendes Deckvermögen bzw. deutlich abweichende Farbtöne und Farbsättigungswerte auf.

EP 2 999 752 B1 offenbart Metalleffektpigmente auf Basis von dünnen Al-Substratplättchen aus einem VMP-Prozess mit hohem Deckvermögen und Fe₂O₃ als farbgebender Schicht. WO 2013/175339 offenbart Aluminium-basierte Metalleffektpigmente mit mindestens einer Metalloxidschicht. EP 2 303 969 B1 offenbart die Herstellung von Mischungen aus Eisen und Aluminiumeffektpigmenten. WO 2015/040537 A1 offenbart nicht magnetisierbare Effektpigmente bestehend aus einem Aluminium oder Aluminiumlegierungs-Kern mit einer oder mehreren Passivierungsschichten und einer Aluminium dotierten Eisenoxidschicht. WO 2007/093334 A1 offenbart ein Verfahren zur Herstellung farbiger Effektpigmente und deren Verwendung in Kosmetika. Die farbigen Effektpigmente bestehen dabei aus einem aluminiumhaltigen Kern, umgeben von einer farbgebenden Schicht, welche durch nasschemische Oxidation des AI-Kerns erzeugt wird, und/oder ein Effektpigment mit mindestens einer Metalloxidschicht, welche mindestens ein Farbpigment enthält. CA 2,496,126 offenbart Effektpigmente auf Metallbasis (Silber, Gold, Kupfer, Aluminium, Zink, Titan und Legierungen daraus) für die Verwendung in Kosmetika, die in einem nasschemischen Verfahren über einen Sol-Gel Prozess mit einer weiteren Metalloxid-Lage beschichtet werden. US 7,828,890 B2 offenbart Effektpigmente mit einem Aluminium- oder Aluminiumlegierungskern und einer den Aluminium- oder Aluminiumlegierungskern umhüllenden Aluminiumoxid- oder Aluminiumoxid/hydroxid-haltigen Schicht, erhältlich durch naßchemische Oxidation plättchenförmiger Aluminium- oder Aluminiumlegierungs-Pigmente, wobei der Gehalt an metallischem Aluminium im Aluminium- oder Aluminiumlegierungskern nicht mehr als 90 Gew.-%, bezogen auf das Gesamtgewicht des Pigments, beträgt, wobei die oxidierten Aluminium- oder Aluminiumlegierungs-Pigmente wenigstens eine hochbrechende Metallchalkogenidschicht mit einem Brechungsindex von > 1,95 aufweisen und zwischen der hochbrechenden Metallchalkogenidschicht und der umhüllenden Aluminiumoxid- oder Aluminiumoxid/hydroxid-haltigen Schicht eine Mischschicht ausgebildet ist. EP 0 950 693 A1 offenbart mehrschichtige Perlglanzpigmente auf Basis eines nichtmetallischen Substrats. WO 2011/085780 A1 offenbart mehrschichtige Effektpigmente auf Basis nichtmetallischer Substrate. WO 96/22336 A1 offenbart farbige Pigmente auf Basis von plättchenförmigen, mit einer Metalloxidschicht beschichteten Aluminiumsubstraten mit Farbpigmenten in der Metalloxidschicht. In dem Fachbuch "Metalleffekt-Pigmente" (2. Überarb. Aufl., Peter Wißling et al., Vincentz-Verlag) wird in Teil III - Spezielle Aluminiumpigmente in Kapitel 2 - Farbige Aluminiumpigmente (S.70-76) die Herstellung eines oxidierten Aluminiumpigments mit "champagnerfarbener" Optik (Handelsname ALOXAL ®) und die Verwendung desselben in Kombination mit farbigen Pigmenten dargestellt. Analog dazu offenbart DE 195 20 312 B4 die Herstellung farbiger Effektpigmente durch nasschemische Oxidation von Aluminium-Plättchen.

Die Aufgabe der vorliegenden Erfindung ist es somit, Metalleffektpigmente mit hohem Deckvermögen und einem gelblich-beigen Farbton mit einem Bunttonwinkel (hᵤᵥ) im Bereich von 50-65 (Champagner-Farbton) und einer Sättigung (Sᵤᵥ) im Bereich von 0,2-0,3 (bei einem ΔE45° von ≤ 3) bereitzustellen.

Diese Aufgabe wird durch die in den Ansprüchen gekennzeichneten Ausführungsformen gelöst.

Insbesondere wird ein metallisches Effektpigment auf der Basis von beschichteten Aluminium-Substratplättchen bereitgestellt, wobei die Aluminium-Substratplättchen eine Dicke von 5 bis 35 nm, vorzugsweise von 15 bis 25 nm, aufweisen, monolithisch aufgebaut sind und von einer "nativen", d.h. durch den Herstellungsprozess bedingten, aber nicht in einem separaten Prozessschritt aufgebrachten, Passivierungsschicht aus Al₂O₃ von weniger als 10 nm, üblicherweise 2 bis 5, in der Regel 2 bis 3 nm umgeben sind, wobei diese Aluminium-Substratplättchen von mindestens einer Beschichtung A aus SiO₂ mit einer Schichtdicke von 10 bis 100 nm, bevorzugt 20 bis 80 nm, umhüllt sind, worauf eine weitere, die Substratplättchen mit der Beschichtung A aus SiO₂ umhüllende Beschichtung B aus Fe₂O₃ mit einer Schichtdicke von 5 bis 45 nm, bevorzugt 10 bis 45 nm, mehr bevorzugt 20 bis 45 nm, insbesondere 20 bis 40 nm aufgebracht ist.

Gegebenenfalls kann noch eine weitere, äußere Schicht C vorgesehen werden, die wiederum die Beschichtung B umhüllt, beispielsweise eine 1 bis 30 nm dicke SiO₂-Schicht, die gegebenenfalls auch die untere Fe₂O₃ durchdringen kann. Zudem kann auf die Schicht B oder auf eine solche Schicht C, wenn vorhanden, eine organische oder organisch-anorganische Oberflächenmodifizierung aufgebracht werden.

Die Schicht A aus SiO₂, welche als eigentliche Passivierungsschicht angesehen werden kann, wird dabei nasschemisch über einen Sol-Gel Prozess aufgebracht. Die Schicht B besteht aus nasschemisch über eine Fällungsreaktion aufgebrachtem Fe₂O₃ mit einer Schichtdicke von 5 bis 45 nm, bevorzugt 10 bis 45 nm, mehr bevorzugt 20 bis 45 nm, insbesondere 20 bis 40 nm. Die Eisenoxidschicht kann zudem eine Mischschicht sein, die eines oder beide der Metalloxide, ausgewählt aus Al₂O₃, oder TiO₂, in einem (Gesamt)anteil von mehr als 0 bis 20 Gew.-%, bezogen auf die Schicht B, enthält.

Die Aluminium-Substratplättchen sind polykristallin und weisen eine durchschnittliche Dicke von 5 bis 35 nm, vorzugsweise von 15 bis 25 nm, auf. Vorzugsweise weist jedes Substratplättchen eine möglichst einheitliche Dicke auf. Herstellungsbedingt können allerdings Schwankungen der Dicke innerhalb eines Plättchens auftreten. Diese sollten vorzugsweise nicht mehr als ±25%, bezogen auf die durchschnittliche Dicke des betrachteten Plättchens, besonders bevorzugt höchstens ±10%, betragen. Unter der durchschnittlichen Dicke ist hier das Zahlenmittel aus maximaler und minimaler Dicke zu verstehen. Die Ermittlung der minimalen und maximalen Schichtdicke erfolgt durch Ausmessen auf Grundlage transmissions- und rasterelektronenmikroskopischen (TEM und REM) Aufnahmen beschichteter Substratplättchen.

Als Aluminiumplättchen werden vorzugsweise sog. *vacuum metallized pigments* (VMP) verwendet. VMPs können durch das Ablösen von Aluminium von metallisierten Folien gewonnen werden. Sie zeichnen sich durch eine besonders geringe Dicke der Substratplättchen und durch eine besonders glatte Oberfläche mit erhöhter Reflektivität aus. Unter Plättchen bzw. Flakes werden solche im Rahmen der vorliegenden Erfindung verstanden, die ein Dicken-/Längenverhältnis von mindestens 1:100, vorzugsweise darüber, aufweisen.

Soweit hier auf die "Dicke" einer Beschichtung oder eines Aluminium-Substratplättchens Bezug genommen wird, gilt damit die durchschnittliche Dicke als bezeichnet, sofern an betreffender Stelle nichts anderes bestimmt ist.

Die Aluminium-Substratplättchen sind monolithisch aufgebaut. Monolithisch bedeutet in diesem Zusammenhang aus einer einzigen abgeschlossenen Einheit ohne Brüche, Schichtungen oder Einschlüsse bestehend, wobei jedoch innerhalb der Substratplättchen Gefügewechsel auftreten können. Die Aluminium-Substratplättchen sind vorzugsweise homogen aufgebaut, d.h. dass innerhalb der Plättchen kein Konzentrationsgradient auftritt. Insbesondere sind die Aluminium-Substratplättchen nicht schichtartig aufgebaut (die "native", d.h. durch den Herstellungsprozess bedingte, aber nicht in einem separaten Prozessschritt aufgebrachte, Passivierungsschicht aus Al₂O₃ wird hier nicht berücksichtigt) und weisen keine darin verteilten Teilchen oder Partikel auf. Sie weisen insbesondere keinen Kern-Schale Aufbau auf, wobei die Schale beispielsweise aus einem für die Substratplättchen geeigneten Material und der Kern aus einem anderen Material, beispielsweise einem Siliciumoxid, besteht. Im Gegensatz dazu bedingt ein komplexerer, nicht-monolithischer Aufbau der Substratplättchen einen erschwerten, zeit- und kostenintensiven Herstellungsprozess.

Die Prozentangaben in den nachstehenden Abschnitte sind, sofern nicht anders vermerkt, als Gew.-% zu lesen.

Die erfindungsgemäßen Metalleffektpigmente zeigen für (deckende) Rakelungen mit ΔE45°≤1 Werte für die Sättigung (Sᵤᵥ) im Bereich von 0,2-0,3, Werte für den Bunttonwinkel (hᵤᵥ) im Bereich von 50-65, Werte für den Flop/Alman Index über 26 und Werte für den Gdiff (Körnigkeit/Graininess) bei maximal 6. Das sehr gute Deckvermögen der erfindungsgemäßen Metalleffektpigmente zeigt sich darin, dass für deckende Rakelungen, die hier mit einem ΔE45°≤1 definiert sind, Pigmentierungen von maximal 7 % benötigt werden. Insbesondere liegen die Werte für ΔE45° für Pigmentierung von 7 % unterhalb von 3,0. Ein derartiger Farbton mit entsprechendem Deckvermögen und Flop kann nicht durch Mischungen von Metalleffektpigmenten mit organischen und/oder anorganischen Pigmenten erzielt werden. Zudem sind Pigmentmischungen weniger präzise in der Reproduktion der Farbtoneinstellung.

Die erfindungsgemäßen Metalleffektpigmente sind, im Gegensatz zu Pigmentmischungen, die auf Aluminiumpigmenten basieren, nicht brennbar und daher z.B. für den Transport nicht als Gefahrgut einzustufen.

Demgegenüber erreicht beispielsweise das nicht erfindungsgemäße Beispiel des im Handel erhältlichen Pigments Merck Iriodin 9602 die oben genannten Werte für das Deckvermögen ΔE45° auch bei hohen Pigmentierungen nicht. Ein Deckvermögen mit einem Wert von ΔE45°<3 kann auch bei sehr hohen Pigmentierungen von 20% (ΔE45 ist hier 5,1) nicht erreicht werden. Eine deckende Rakelung mit ΔE45°≤1 kann rechnerisch erst bei einer Pigmentierung von >30% erwartet werden.

Wenn das im Handel erhältliche Pigment Iriodin 9602 mit einer Pigmentierung von 20% mit einem Gelbpigment Helio Beit UN210 (4,5%; Einwaagen des Gelb-Pigments, sofern nicht anders gekennzeichnet, bezogen auf die Trockenmasse des verwendeten Pigments) in dem Versuch, einen Champagner-Farbton nachzustellen, gemischt wird, liegt der Wert des Deckvermögens ΔE45° dieser Mischung im oberen Bereich des von den erfindungsgemäßen Metalleffektpigmenten erreichten Bereichs, allerdings ist hier die deutlich höhere Pigmentierung zu beachten. Der Wert für die Sättigung der Mischung liegt im von den erfindungsgemäßen Metalleffektpigmenten erreichten Bereich von 0,2-0,3, allerdings kann mit einer solchen Zusammensetzung der von den erfindungsgemäßen Metalleffektpigmenten erreichte Bunttonwinkel (hᵤᵥ) und der definierte Flop-Wert (hier erhöht der Zusatz der Gelbpaste das Deckvermögen gegenüber dem reinen Pigment, allerdings verschlechtert sich durch den Zusatz der Flop/Alman-Index) nicht erzielt werden.

Es kann auch ein Silberdollar-Pigment im Handel erhältlich von Schlenk Metallic Pigments GmbH unter der Bezeichnung "Alustar 10200", mit 4%iger Pigmentierung mit 4,5% (Einwaagen des Gelb-Pigments, sofern nicht anders gekennzeichnet, bezogen auf die Trockenmasse des verwendeten Pigments) eines Gelbpigments (Helio Beit UN120), im Handel erhältlich von Helio Beit Pigmentpasten GmbH, gemischt werden, um einen Farbton im Bereich der erfindungsgemäßen Pigmente zu erhalten. Das Deckvermögen ΔE45° einer so erhaltenen Mischung liegt für beide zwar im von den erfindungsgemäßen Metalleffektpigmenten erreichten Bereich. Flop und Gdiff der Mischung liegen ebenfalls im Bereich der erfindungsgemäßen Metalleffektpigmente. Allerdings können mit einem Wert für die Sättigung (Sᵤᵥ) von ca. 0,5 und einem Wert von ca. 70 für den Bunttonwinkel (hᵤᵥ) die Bereiche der erfindungsgemäßen Metalleffektpigmente nicht erzielt werden.

Wird in der vorstehenden Mischung der Anteil des Gelbpigments von 4,5% auf 10% erhöht, kann zwar der Bereich für den Bunttonwinkel (hᵤᵥ) der erfindungsgemäßen Metalleffektpigmente getroffen werden, allerdings führt dies zu einer höheren Sättigung, die deutlich außerhalb des Bereichs der erfindungsgemäßen Metalleffektpigmente liegt.

Ein gelblich-beiger Champagner-Farbton mit einem Bunttonwinkel (hᵤᵥ) im Bereich von 50-65 mit sehr hohem Deckvermögen und einer Sättigung (Sᵤᵥ) im Bereich von 0,2-0,3 kann in dieser Kombination nicht über Mischungen von Perlglanzpigmenten und/oder Metalleffektpigmenten untereinander oder durch Zusatz von Carbon Black oder Farbstoffen erzeugt werden. Bei annähernd gleichem Farbton treten auch Unterschiede im Flop auf. Häufig muss Carbon Black für eine Verbesserung des Deckvermögens zugesetzt werden.

Weitere Nachteile von Mischungen von silbernen Pigmenten z.B. auf Basis von Silverdollars mit organischen Farbstoffen, z.B. mit einem organischen Gelbpigment sind wie folgt: UV- bzw. Temperaturstabilitäten sind geringer, ein zusätzlicher Mischvorgang ist nötig, unter Umständen sind Zusätze von Stabilisatoren oder Emulgatoren nötig. Einfärbungen von Al-Pigmenten mit den UV-Stabilieren anorganischen (Gelb-)Pigmenten erreichen nicht die entsprechende Brillanz eines Al-Pigments mit einer entsprechenden Eisenoxid-Schicht (Literatur zur Photochemischen Zersetzung organischer Pigmente: Lisa Ghelardi, Ilaria Degano, Maria Perla Colombini, Joy Mazurek, Michael Schilling, Herant Khanjian, Tom Learner, A multi-analytical study on the photochemical degradation of synthetic organic pigments, Dyes and Pigments, Volume 123, 2015, Pages 396-403).

Des Weiteren lässt sich der gewünschte Farbton mit einer Sättigung (Sᵤᵥ) im Bereich von 0,2-0,3 nicht durch Mischung eines goldfarbenen Aluminiumpigments (beispielhaft ausgewählt das Pigment 21YY (Zenexo Goldenshine 21 YY von Schlenk Metallic Pigments GmbH), das folgenden Pigmentaufbau aufweist: Substrat = Decomet (VMP Aluminiumplättchen) mit Schichtdicke ∼ 25 nm, darauf eine SiO₂-Schicht mit ∼ 60 nm und abschließend eine Fe₂O₃-Schicht mit ∼ 100 nm, mit SiO₂-Nachbeschichtung von ∼ 10 nm) mit einem Ruß, TiO₂ oder einem Perlglanzpigment (hier im Bsp. Iriodin 119 Polar White von der Firma Merck) erreichen. Diese Formulierungen weisen zwar ein gutes Deckvermögen und einen guten Flop auf, der vorstehende Wert für die Sättigung (Sᵤᵥ) kann jedoch über die entsprechenden Abmischungen nicht erreicht werden.

Die Bestimmung des Deckvermögens erfolgt durch Variation der Pigmentierung der Lackansätze und Ermittlung der für eine deckende Rakelung bei vorgegebener Nassfilmdicke benötigten Pigmentierung. Die Pigmentierung (in %) bezieht sich hierbei auf den Anteil der Masse des reinen Pigments an der Gesamtmasse des Lackansatzes in einem lösemittelbasierten Nitrocellulose /Polycyclohexanon/ Polyacryl-Lack (FK∼10%). Dazu werden mindestens vier verschiedene Pigmentierungen mit einer Nassfilmdicke von 38 µm auf einem Schwarz/Weiß-Karton der Firma TQC mittels eines Filmziehgeräts der Firma Zehnter aufgebracht, getrocknet und anschließend der ΔE45° mit einem Gerät Byk-mac I der Firma Byk vermessen. Aus den erhaltenen ΔE45°-Werten wird eine Kurve gefittet und der Wert für die Pigmentierung für einen ΔE45° = 1 ermittelt. Als deckend wird eine Schicht definiert, die einen ΔE45° von ≤ 1 bei einer vorgegeben Nassfilmdicke von 38 µm aufweist. Je geringer die benötige Pigmentierung um diesen Wert zu erreichen, desto höher das Deckvermögen.

In der Regel haben die Plättchen mittlere größte Durchmesser von etwa 2 bis 200 µm, insbesondere etwa 5 bis 100 µm. Der d₅₀ der erfindungsgemäßen Pigmente (Al-Substrat+SiO₂+Fe₂O₃-Schicht) liegt in der Regel bei 15-30 µm. Hierin wird der d₅₀-Wert, soweit nicht anders angegeben, mit einem Gerät des Typs Sympatec Helos mit Quixel-Nassdispergierung bestimmt.

Allgemein ist eine Beschichtung eines Teils der Oberfläche der beschichteten Substratplättchen ausreichend, um ein Glanzpigment zu erhalten. So können beispielsweise lediglich die obere und/oder untere Seite der Plättchen beschichtet sein, wobei die Seitenfläche(n) ausgespart sind. Erfindungsgemäß ist allerdings die gesamte Oberfläche der gegebenenfalls passivierten Substratplättchen, einschließlich der Seitenflächen, von Beschichtung A und B bedeckt. Die Substratplättchen sind also vollständig von der Beschichtung A und B umhüllt. Dies verbessert die optischen Eigenschaften des erfindungsgemäßen Pigments und erhöht die mechanische und chemische Belastbarkeit der beschichteten Substratplättchen.

Das erfindungsgemäße Verfahren zur Herstellung des metallischen Glanzpigments umfasst die Schritte des Bereitstellens von in der Regel passivierten Aluminium-Substratplättchen, das Aufbringen der Schicht A aus SiO₂ nasschemisch über einen Sol-Gel Prozess auf ein solches zuvor bereitgestelltes Aluminium-Substratplättchen und anschließend das Aufbringen der Schicht B aus Fe₂O₃ mittels nasschemischer Fällung eines oder mehrerer Eisen(IIl)salze und nachfolgender Kalzinierung.

Zur Erzeugung von Beschichtung A werden zweckmäßigerweise organische Siliciumverbindungen, bei denen die organischen Reste über Sauerstoffatome an die Metalle gebunden sind, in Gegenwart der Substratplättchen hydrolysiert. Bevorzugtes Beispiel für die organischen Siliciumverbindungen ist Tetraethoxysilan (Tetraethylorthosilikat, TEOS). Die Hydrolyse wird vorzugsweise in Gegenwart einer Base, z.B. KOH, NaOH, Ca(OH)₂ oder NH₃, oder einer Säure, beispielsweise Phosphorsäure und organische Säuren wie Essigsäure, als Katalysator durchgeführt. Wasser muss mindestens in der stöchiometrisch für die Hydrolyse erforderlichen Menge vorliegen, bevorzugt ist jedoch die 2 bis 100-fache, insbesondere die 5 bis 20-fache Menge. Bezogen auf die eingesetzte Wassermenge gibt man in der Regel 1 bis 40 Vol.-%, vorzugsweise 1 bis 10 Vol.-%, einer 25 Gew.-%igen wässrigen Base z.B. KOH, NaOH, Ca(OH)₂ oder NH₃ zu. Für die Temperaturführung hat es sich als vorteilhaft erwiesen, das Reaktionsgemisch für 1-12 h, bevorzugt für 2-8 h auf 40-80°C, bevorzugt auf 50-70°C zu erhitzen.

Verfahrenstechnisch erfolgt das Beschichten von Substratplättchen mit einer Beschichtung A zweckmäßigerweise wie folgt:
Aluminium-Substratplättchen, organisches Lösungsmittel, Wasser und Katalysator werden vorgelegt, wonach die zu hydrolysierende Siliciumverbindung, als Reinstoff oder gelöst, z.B. als 30 bis 70, bevorzugt 40 bis 60 Vol.-%ige Lösung im organischen Lösemittel, zum vorgeheizten Reaktionsgemisch zugegeben wird. Die Siliciumverbindung kann aber auch bei erhöhter Temperatur kontinuierlich zudosiert werden, wobei Wasser und wässrige Base vorgelegt oder ebenfalls kontinuierlich zudosiert werden können. Nach beendeter Beschichtung wird das Reaktionsgemisch wieder auf Raumtemperatur abgekühlt.

Um eine Agglomeratbildung während des Beschichtungsvorgangs zu verhindern, kann die Suspension einer starken mechanischen Beanspruchung wie Pumpen, kräftigem Rühren oder Einwirken von Ultraschall unterzogen werden.

Gegebenenfalls kann der Beschichtungsschritt ein- oder mehrfach wiederholt werden. Sollte die Mutterlauge milchig trüb aussehen, so empfiehlt es sich, diese vor einer weiteren Beschichtung auszutauschen.

Die Isolierung der mit Beschichtung A ummantelten Aluminium-Substratplättchen kann in einfacher Weise durch Abfiltrieren, Waschen mit organischem Lösungsmittel, vorzugsweise den auch als Lösemittel üblicherweise verwendeten Alkoholen, und anschließendes Trocknen (üblicherweise 2 bis 24 h bei 20 bis 200 °C) erfolgen.

Das Aufbringen der Schicht B kann durch hydrolytische Zersetzung von Eisen(III)salzen wie Eisen (III)-chlorid und -sulfat und anschließendes Überführen der gebildeten hydroxidhaltigen Schicht durch Kalzinieren in die Oxidschicht aufgebracht werden. Das Tempern erfolgt vorzugsweise bei einer Temperatur von 250 bis 550°C für eine Dauer von 5 bis 360 Minuten, vorzugsweise 300 bis 450°C für eine Dauer von 30 bis 120 Minuten.

Wenn die Eisenoxidschicht als eine Mischschicht vorgesehen wird, die eines oder beide der Metalloxide, ausgewählt aus Al₂O₃, oder TiO₂, in einem (Gesamt)anteil von 0 bis 20 Gew.-%, bezogen auf die Schicht B, enthält, können neben dem Eisen(III)salz beispielsweise AlCl₃ bzw. TiOCl₂ als Vorläuferverbindungen für Al₂O₃ bzw. TiO₂ eingesetzt werden.

Mit Hilfe des erfindungsgemäßen Herstellungsverfahrens können die beschichteten Substratplättchen in einfacher Weise in großen Mengen reproduzierbar hergestellt werden. Es werden vollständig umhüllte Pigmentteilchen mit hoher Qualität der einzelnen Beschichtungen (homogen, filmartig) erhalten.

Die erfindungsgemäßen Metalleffektpigmente lassen sich vorteilhaft in Kosmetika verwenden, insofern sie toxikologisch/mikrobiologisch unbedenklich sind. Ein Einsatz ist hier in großer Bandbreite möglich: hauptsächlich Nagellacke, Lipgloss, Lidschatten, Lippenstiften. Weitere Verwendungsmöglichkeiten ergeben sich z.B. in Shampoos und Pudern.

Zur Verstärkung des Effekts kann das erfindungsgemäße Pigment mit organischen oder anorganischen Farbstoffen gemischt werden.

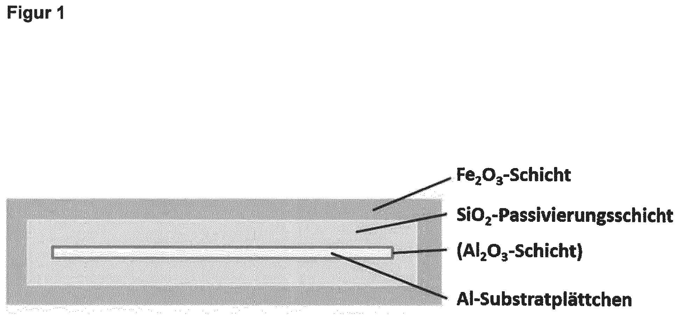

Figur 1 zeigt den allgemeinen Schichtaufbau der erfindungsgemäßen Metalleffektpigmente (nicht maßstabsgetreu).

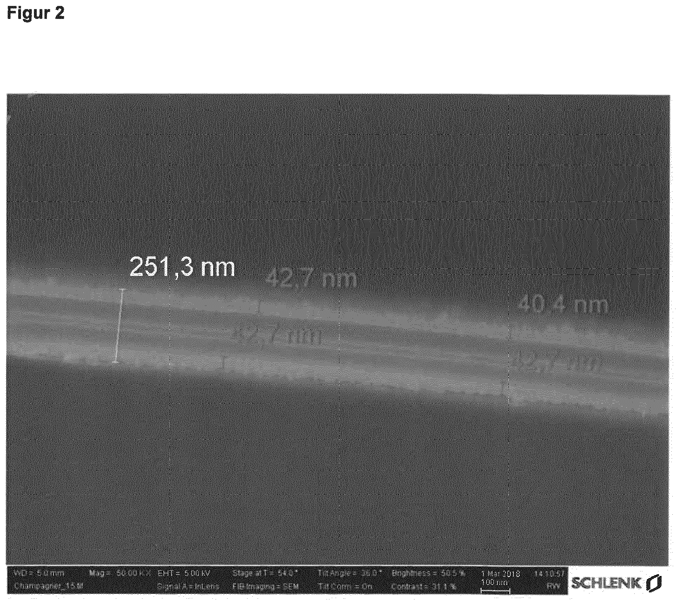

Figur 2 zeigt den REM-Schnitt eines erfindungsgemäßen Metalleffektpigments, nämlich das erfindungsgemäße Pigment (Aufbau dieses Pigments: Substrat = Decomet (VMP Aluminiumplättchen) mit Schichtdicke ∼ 25 nm, darauf eine SiO₂-Schicht mit ∼ 60 nm und abschließend eine Fe₂O₃-Schicht mit ∼ 45 nm, ohne eine Nachbeschichtung, wie z.B. eine SiO₂-Nachbeschichtung, und ohne Oberflächenmodifizierung.

Die nachstehenden Beispiele dienen als weitere Erläuterung der vorliegenden Erfindung, ohne darauf beschränkt zu sein.

### Beispiele

Erfindungsgemäßes Pigment = Decomet (VMP Aluminium) als Substrat, Schicht A= SiO₂ mit Schichtdicke im erfindungsgemäßen Bereich, Schicht B= Fe₂O₃ mit Schichtdicke im erfindungsgemäßen Bereich.

### Erfindungsgemäße Beispiele

(1) Erfindungsgemäßes Pigment + ca. 1,6% Al₂O₃
(2) Erfindungsgemäßes Pigment
(3) Erfindungsgemäßes Pigment mit ca. 6% TiO₂
(4) Erfindungsgemäßes Pigment mit ca. 1,6% Al₂O₃ + SiO₂ (Schicht C, rechn. Dicke 16,5 nm)
(5) Erfindungsgemäßes Pigment mit ca. 1,6% Al₂O₃ + SiO₂ (Schicht C, rechn. Dicke 26,5 nm)

### Nicht erfindungsgemäße Beispiele

(6) Merck Iriodin 9602 mit 4,5 % Gelbpigment Helio Beit UN 210
(7) Merck Iriodin 9602
(8) Alustar 10200 mit 4 % Gelbpigment Helio Beit UN 210
(9) Alustar 10200 mit 10 % Gelbpigment Helio Beit UN 210
(10) Pigment 21 YY (Zenexo GoldenShine, Schlenk Metallic Pigments GmbH)
(11) Pigment 21YY mit 1% Helio Beit Carbon Black (Rußdispersion)
(12) Pigment 21YY mit 10% Iriodin 119
(13) Pigment 21YY mit 20 % Iriodin 119
(14) Pigment 21YY mit 50 % Iriodin 119
(15) Pigment 21YY mit 1,5 % TiO₂

### Herstellung von erfindungsgemäßen Beispielen

Zunächst wurden Al-Plättchen mittels Sol-Gel-Verfahren unter Verwendung von Tetraethylorthosilikat (TEOS) mit SiO₂ beschichtet (vgl. beispielsweise WO 2015/014484 A1).

Anschließend wird in ein 2L-Becherglas die Menge an Suspension vorgelegt, die einem Aluminumgehalt von 15 g entspricht. Es wird mit Leitungswasser auf 900 g aufgefüllt. Zu Beginn der Synthese wird leicht angesäuert. Anschließend beginnt die Zugabe der FeCl₃-Lösung, der pH-Wert wird durch Zugabe einer alkalischen Lösung, z.B. KOH, NaOH, Ca(OH)₂ oder NH₃, konstant gehalten. Am Ende der Synthese wird der pH-Wert unter Rühren angehoben. Anschließend wird nach einer Sedimentation das erhaltene Pigment über Filtration abgetrennt getrocknet und anschließend kalziniert.

Die nachstehende Tabelle gibt die Schichtdicken und koloristischen Daten vorstehender erfindungsgemäßer und nicht erfindungsgemäßer Beispiele an:

| **Bezeichnung der Beispiele** | **Nr.** | **Bunttonwinkel (hᵤᵥ)** | **Sättigung (Sᵤᵥ)** | **Deckvermögen ΔE45° (% Pigmentierung)** | **% Pigmentierung für ΔE45°=1** | **Alman-Index** | **Gdiff** | **erfindungsgemäß** |
|---|---|---|---|---|---|---|---|---|
| **erfindungsgemäßer Bereich** | | **50-65** | **0,2-0,3** | **≤ 3 (7%)** | **< 7%** | **> 26** | **< 6** | |
| Erfindungsgemäßes Pigment (mit ca.1,6 % Al₂O₃) | 1 | 55-57 | 0,23-0,24 | 3,4 (4,2%), 0,06 (7,2%) | 5,1 | 33-34 | 4,0-5,0 | ja |
| Erfindungsgemäßes Pigment | 2 | 54 | 0,26 | 0,2 (7%) | 5,5 | 34 | 5,0 | ja |
| Erfindungsgemäßes Pigment (mit ca. 6,0 % TiO₂) | 3 | 61 | 0,20 | 2,4 (5%), 0,2 (7%) | 5,7 | 34 | 4,7-5,3 | ja |
| Erfindungsgemäßes Pigment (mit ca. 1,6 % Al₂O₃) +16.5 nm (errechnet) SiO₂ (Schicht C) | 4 | 56 | 0,26 | 2,4 (5%) | 6,2 | 33,6 | 5,2 | ja |
| Erfindungsgemäßes Pigment (mit ca. 1,6 % Al₂O₃) +26.5 nm (errechnet) SiO₂ (Schicht C) | 5 | 56 | 0,26 | 2,8(5%) | 6,7 | 33,2 | 5,3 | ja |
| Merck Iriodin 9602 + 4,5% Gelbpigment Helio Beit UN 210 | 6 | 71 | 0,26 | 8,4 (20%) | - | 22 | 3,8 | nein |
| Merck Iriodin 9602 | 7 | 238-239 | 0,10-0,13 | 54 (4,2%), 35 (7,2%) | 31,8 | 27-31 | 5,0-6,5 | nein |
| Alustar 10200 + 4% Gelbpigment Helio Beit UN 210 | 8 | 71 | 0,48 | 1,6 (4%) | - | 28 | 5,4 | nein |
| Alustar 10200 + 10% Gelbpigment Helio Beit UN 210 | 9 | 65 | 0,70 | 1,2 (4%) | - | 26 | 5,5 | nein |
| 21 YY (Zenexo GoldenShine) | 10 | 62 | 0,92 | 1,8 (7%) | 7,8 | 31 | 5,0 | nein |
| 21 YY (Zenexo GoldenShine) + 1 % Helio Beit Carbon Black | 11 | 63 | 0,89 | 2,2 (7%) | - | 31 | 5,2 | nein |
| 21 YY (Zenexo GoldenShine) + Merck Iriodin 119 10% | 12 | 63 | 0,83 | 3,1 (7%) | 9,1 | 29 | 5,1 | nein |
| 21 YY (Zenexo GoldenShine) + Merck Iriodin 119 20% | 13 | 63 | 0,75 | 4,4 (7%) | 9,6 | 27 | 5,0 | nein |
| 21 YY (Zenexo GoldenShine) + Merck Iriodin 119 50% | 14 | 62 | 0,50 | 10,2 (7%) | 13,2 | 22 | 4,7 | nein |
| 21 YY (Zenexo GoldenShine) + TiO₂ 1,5 % | 15 | 63 | 0,89 | 2,4 (7%) | - | 28 | 5,1 | nein |

- Die REM-Aufnahmen wurden jeweils mit einem Rasterelektronenmikroskop Zeiss Auriga 40 aufgenommen.
- Die Koloristischen Daten wurden durch Vermessen von Rakelungen der Pigmente (sofern nicht anders vermerkt mit einer Pigmentierung von 7% in einem lösemittelbasierten Nitrocellulose/Polycyclohexanon/Polyacryl-Lack, FK-Anteil ∼ 10%)) auf einem DIN A5 schwarz-weiß-Karton der Firma TQC mit einer 38µm Spiralrakel auf einem automatischen Filmziehgerät der Firma Zehntner hergestellt.
- Die Vermessung der koloristischen Daten erfolgte mit dem Byk-mac I der Firma Byk.

## Patentansprüche

1. Metallisches Effektpigment auf der Basis von beschichteten Aluminium-Substratplättchen, wobei die Aluminium-Substratplättchen eine Dicke von 5 bis 35 nm aufweisen, monolithisch aufgebaut sind und von einer nativen Passivierungsschicht aus Al₂O₃ von weniger als 10 nm umgeben sind, wobei diese Aluminium-Substratplättchen von mindestens einer Beschichtung A aus SiO₂ mit einer Schichtdicke von 10 bis 100 nm umhüllt sind, worauf eine weitere, die Substratplättchen mit der Beschichtung A aus SiO₂ umhüllende Beschichtung B aus Fe₂O₃ mit einer Schichtdicke von 5 bis 45 nm aufgebracht ist.

2. Metallisches Effektpigment gemäß Anspruch 1, wobei die Aluminium-Substratplättchen eine Dicke von 15 bis 25 nm aufweisen.

3. Metallisches Effektpigment gemäß Anspruch 1 oder 2, wobei die Beschichtung A aus SiO₂ eine Schichtdicke von 20 bis 80 nm aufweist.

4. Metallisches Effektpigment gemäß einem der Ansprüche 1 bis 3, wobei die Beschichtung B aus Fe₂O₃ eine Schichtdicke von 10 bis 45 nm aufweist.

5. Metallisches Effektpigment gemäß einem der Ansprüche 1 bis 4, wobei die Eisenoxidschicht eine Mischschicht ist, die eines oder beide der Metalloxide, ausgewählt aus Al₂O₃ oder TiO₂, in einem Anteil von mehr als 0 bis 20 Gew.-%, bezogen auf die Schicht B, enthält.

6. Metallisches Effektpigment gemäß einem der Ansprüche 1 bis 5, gekennzeichnet bei einem ΔE45 von ≤ 3 bei einer Pigmentierung von maximal 7%, insbesondere ΔE45° von ≤ 1 bei einer Pigmentierung von < 7%, durch eine Sättigung (Sᵤᵥ) im Bereich von 0,2-0,3, einen Wert für den Bunttonwinkel (hᵤᵥ) im Bereich von 50-65, einen Wert für den Flop/Alman Index über 26 und einen Wert für den Gdiff (Körnigkeit/Graininess) bei maximal 6.

7. Metallisches Effektpigment gemäß einem der Ansprüche 1 bis 6, weiter umfassend eine äußere Schicht C, die wiederum die Beschichtung B umhüllt, insbesondere eine 1 bis 30 nm dicke SiO₂-Schicht, die gegebenenfalls auch die untere Fe₂O₃ durchdringen kann.

8. Metallisches Effektpigment gemäß einem der Ansprüche 1 bis 7, wobei auf die Schicht B oder auf die Schicht C, wenn vorhanden, eine organische oder organisch-anorganische Oberflächenmodifizierung aufgebracht worden ist.

9. Verfahren zur Herstellung des metallischen Effektpigments gemäß einem der Ansprüche 1 bis 8, umfassend das Aufbringen der Schicht A aus SiO₂ nasschemisch über einen Sol-Gel Prozess auf ein zuvor bereitgestelltes Aluminium-Substratplättchen und anschließend das Aufbringen der Schicht B aus Fe₂O₃ mittels nasschemischer Fällung eines oder mehrerer Eisen(III)salze und nachfolgender Kalzinierung.

10. Verwendung der metallischen Effektpigmente nach einem der Ansprüche 1 bis 8 zur Einfärbung von Lacken, Druckfarben, Tinten, Kunststoffen, Gläsern, keramischen Produkten und Zubereitungen der dekorativen Kosmetik, insbesondere in Nagellacken, Lipgloss, Lidschatten, Lippenstiften, Shampoos bzw. Pudern, wobei die metallischen Effektpigmente alleine oder in Mischungen mit organischen und/oder anorganischen Pigmenten verwendet werden können.
